# EUROPEAN PATENT APPLICATION

(11) **EP 4 450 171 A1**
(43) Date of publication of application: **23.10.2024**
(21) Application number: 23745948.2
(22) Date of filing: 10.01.2023
(51) Int. Cl.: B05B 17/06, A24F 47/00

(54) **ULTRASONIC ATOMIZER AND WORKING STATE DETERMINING METHOD THEREOF**

(30) Priority: 26.01.2022 CN 202210094558
(71) Applicant: Shenzhen First Union Technology Co., Ltd., Shenzhen, Guangdong 518000 (CN)
(72) Inventor: LI, Xinjun, Shenzhen, Guangdong 518000 (CN); XU, Zhongli, Shenzhen, Guangdong 518000 (CN); LI, Yonghai, Shenzhen, Guangdong 518000 (CN)
(74) Representative: Ran, Handong
(86) International application number: PCT/CN2023/071642
(87) International publication number: WO 2023/143065

(57) **Abstract**

An ultrasonic atomizer and a working state determining method thereof. The ultrasonic atomizer (100) comprises: a liquid storage cavity (11), an ultrasonic atomization sheet (12), a controller (13), a control circuit (14), and a power supply (15). The control circuit (14) comprises: a first switch branch (141), which is turned on or off in response to a first pulse signal outputted by the controller (13); a first boost branch (142), which boosts an output voltage of the power supply (15) in response to turning-on or -off of the first switch branch (141), so as to output a first drive signal for driving the ultrasonic atomization sheet (12); and an energy storage branch (143), which, in response to the first drive signal, stores energy and outputs a first detection voltage to the controller (13), so that the controller (13) determines a current working state of the ultrasonic atomizer (100) according to the first detection voltage. The ultrasonic atomizer can reduce the risk of damage.

## Description

### CROSS-REFERENCE TO RELATED APPLICATIONS

This application claims priority to Chinese Patent Application No. 202210094558.5, filed with the China National Intellectual Property Administration on January 26, 2022 and entitled "ULTRASONIC ATOMIZER AND WORKING STATE DETERMINING METHOD THEREOF", which is incorporated herein by reference in its entirety.

### TECHNICAL FIELD

This application relates to the field of ultrasonic atomizer technologies, and in particular, to an ultrasonic atomizer and a working state determining method thereof.

### BACKGROUND

In daily life, an ultrasonic atomizer may be used in a plurality of fields, such as humidification, aroma diffusion, sterilization, decoration, medical atomization, and electronic cigarettes. The ultrasonic atomizer uses an ultrasonic atomization technology to implement an atomization function. Specifically, in the ultrasonic atomizer, an ultrasonic atomization sheet can convert electrical energy into ultrasonic energy, and the ultrasonic energy can atomize water-soluble atomization liquid into tiny mist particles of 1 µm to 5 µm at normal temperature, so that water can be used as a medium, and the water-soluble atomization liquid can be sprayed into mist using ultrasonic directional pressure.

In related art, in a working process of the ultrasonic atomization sheet, a current of the ultrasonic atomization sheet is usually detected to determine whether the ultrasonic atomizer is in abnormal such as dry burning.

However, for the ultrasonic atomizer, there is a small difference between a current when the ultrasonic atomizer is in abnormal such as dry burning and a current when the ultrasonic atomizer works normally, and consequently, misjudgment is easily caused, resulting in damage to the ultrasonic atomizer caused by an abnormal condition such as dry burning.

### SUMMARY

Embodiments of this application are intended to provide an ultrasonic atomizer and a working state determining method thereof, to reduce a risk of damage to the ultrasonic atomizer.

According to a first aspect, this application provides an ultrasonic atomizer, including:
a liquid storage cavity, configured to store a liquid substrate;
an ultrasonic atomization sheet, configured to generate oscillation to atomize the liquid substrate; and
a controller, a control circuit, and a power supply.

The control circuit includes:
a first switch branch, connected to the controller, where the first switch branch is configured to be turned on or turned off in response to a first pulse signal outputted by the controller;
a first boost branch, separately connected to the power supply, the first switch branch, and the ultrasonic atomization sheet, where the first boost branch is configured to boost an output voltage of the power supply in response to the turning-on or the turning-off of the first switch branch, to output a first drive signal for driving the ultrasonic atomization sheet; and
an energy storage branch, separately connected to the first boost branch, the ultrasonic atomization sheet, and the controller, where the energy storage branch is configured to: store energy in response to the first drive signal, and output a first detection voltage to the controller, so that the controller determines a current working state of the ultrasonic atomizer based on the first detection voltage.

In an optional implementation, the first switch branch includes a first switch.

A first end of the first switch is connected to the controller. A second end of the first switch is connected to the ground. A third end of the first switch is separately connected to the first boost branch and the ultrasonic atomization sheet.

In an optional implementation, the first switch branch further includes a first resistor and a second resistor.

A first end of the first resistor is connected to the controller. A second end of the first resistor is separately connected to the first end of the first switch and a first end of the second resistor. A second end of the second resistor is connected to the ground.

In an optional implementation, the first boost branch includes a first inductor.

A first end of the first inductor is connected to the power supply. A second end of the first inductor is connected to the first switch branch and the ultrasonic atomization sheet.

In an optional implementation, the energy storage branch includes a first capacitor.

A first end of the first capacitor is separately connected to the first boost branch, the first switch branch, and the ultrasonic atomization sheet. A second end of the first capacitor is connected to the ground.

In an optional implementation, a capacitance value of the first capacitor is less than or equal to 100 nF.

In an optional implementation, the control circuit further includes:
a preprocessing branch, separately connected to the first boost branch, the first switch branch, and the ultrasonic atomization sheet, where the preprocessing branch is configured to: perform direct current blocking, voltage division, and filtering on the first drive signal, and output a first drive sub-signal;
a rectifier branch, connected to the preprocessing branch, where the rectifier branch is configured to rectify the first drive sub-signal, so that the energy storage branch stores energy in response to the first drive sub-signal and outputs the first detection voltage; and
a voltage-limiting and current-limiting branch, separately connected to the rectifier branch, the energy storage branch, and the controller, where the voltage-limiting and current-limiting branch is configured to: perform voltage-limiting and current-limiting on the first detection voltage, and output a second detection voltage to the controller, so that the controller determines the current working state of the ultrasonic atomizer based on the second detection voltage.

In an optional implementation, the preprocessing branch includes a second capacitor, a third capacitor, a third resistor, and a fourth resistor.

A first end of the second capacitor is separately connected to the first boost branch, the first switch branch, and the ultrasonic atomization sheet. A second end of the second capacitor is connected to a first end of the third resistor. A second end of the third resistor is separately connected to a first end of the third capacitor, a first end of the fourth resistor, and the rectifier branch. A second end of the third capacitor and a second end of the fourth resistor are connected to the ground.

In an optional implementation, the rectifier branch includes a first diode and a fifth resistor.

An anode of the first diode is connected to the preprocessing branch. A cathode of the first diode is connected to a first end of the fifth resistor. A second end of the fifth resistor is separately connected to the energy storage branch and the voltage-limiting and current-limiting branch.

In an optional implementation, the voltage-limiting and current-limiting branch includes a sixth resistor and a seventh resistor.

A first end of the sixth resistor is separately connected to a first end of the seventh resistor, the rectifier branch, and the energy storage branch. A second end of the sixth resistor is connected to the ground. A second end of the seventh resistor is connected to the controller.

In an optional implementation, the control circuit further includes:
a drive branch, separately connected to the controller, the first switch branch, and the power supply, where the drive branch is configured to output a second pulse signal in response to a current outputted by the power supply and the first pulse signal.

A drive capability of the second pulse signal is stronger than that of the first pulse signal.

In an optional implementation, the drive branch is further configured to output a fourth pulse signal in response to the current outputted by the power supply and a third pulse signal outputted by the controller.

The control circuit further includes:
a second switch branch, connected to the drive branch, where the second switch branch is configured to be turned on or turned off in response to the second pulse signal; and
a second boost branch, separately connected to the power supply, the second switch branch, and the ultrasonic atomization sheet, where the second boost branch is configured to boost the output voltage of the power supply in response to the turning-on or the turning-off of the second switch branch, to output a second drive signal for driving the ultrasonic atomization sheet.

In an optional implementation, the second switch branch includes a second switch, an eighth resistor, and a ninth resistor.

A first end of the second switch is connected to the drive branch. A third end of the second switch is separately connected to the ultrasonic atomization sheet and the second boost branch. A first end of the eighth resistor is connected to the controller. A second end of the eighth resistor is connected to a first end of the ninth resistor. Both a second end of the second switch and a second end of the ninth resistor are connected to the ground.

In an optional implementation, the second boost branch includes a second inductor.

A first end of the second inductor is connected to the power supply. A second end of the second inductor is separately connected to the second switch branch and the ultrasonic atomization sheet.

In an optional implementation, the drive branch includes a drive chip, and the drive chip includes a power supply input end, a first signal input end, a second signal input end, a first signal output end, and a second signal output end.

The power supply input end is connected to the power supply. Both the first signal input end and the second signal input end are connected to the controller. The first signal output end is connected to the first switch branch. The second signal output end is connected to the second switch branch.

The first signal input end is configured to input the first pulse signal. The first signal output end is configured to output the second pulse signal. The second signal input end is configured to input the third pulse signal. The second signal output end is configured to output the fourth pulse signal.

In an optional implementation, the control circuit further includes a current detection branch.

The current detection branch is separately connected to the power supply, the first boost branch, and the controller. The current detection branch is configured to detect a current flowing into the first boost branch.

In an optional implementation, the current detection branch includes an amplifier and a tenth resistor.

The tenth resistor is separately connected to the amplifier, the first boost branch, and the power supply. The amplifier is connected to the controller.

The amplifier is configured to output a third detection voltage based on a voltage across two ends of the tenth resistor, so that the controller determines, based on the third detection voltage, the current flowing into the first boost branch.

According to a second aspect, this application provides a method for determining a working state of an ultrasonic atomizer. The ultrasonic atomizer includes an ultrasonic atomization sheet. The method includes:
obtaining, in a working process of the ultrasonic atomization sheet, a positive half or a negative half of a drive voltage driving the ultrasonic atomization sheet, and performing charge accumulation under driving of the positive half or the negative half of the drive voltage;
obtaining, if a maximum change value of a voltage obtained through the charge accumulation is less than a preset change threshold within first duration, a voltage obtained through current charge accumulation, and recording the voltage as a first detection voltage; and
determining a current working state of the ultrasonic atomizer based on the first detection voltage.

In an optional implementation, the first duration is any one of duration in (0, 10 ms], or the first duration is duration longer than or equal to five sampling periods, and each sampling period is any one of duration in (0, 100 µs].

In an optional implementation, the determining a current working state of the ultrasonic atomizer based on the first detection voltage includes:
determining, if the first detection voltage is less than a first voltage threshold, that the current state of the ultrasonic atomizer is a normal working state; or
determining, if the first detection voltage is greater than or equal to a first voltage threshold, that the current working state of the ultrasonic atomizer is an abnormal working state.

In the ultrasonic atomizer provided in embodiments of this application, in a process in which the ultrasonic atomization sheet is driven by the first drive signal, the energy storage branch may store energy based on the first drive signal to generate the first detection voltage in the energy storage branch. In addition, when the first detection voltage is received, the controller may determine the current working state of the ultrasonic atomizer based on the first detection voltage. For the ultrasonic atomizer, there is a large difference between a voltage when the ultrasonic atomizer is in abnormal such as dry burning and a voltage when the ultrasonic atomizer works normally. Therefore, obtaining the first detection voltage can accurately determine whether the ultrasonic atomizer is in abnormal, so that processing can be performed timely when the ultrasonic atomizer is in abnormal. In this way, a probability that the ultrasonic atomizer is damaged due to an abnormal condition such as dry burning can be reduced. In other words, a risk of damage to the ultrasonic atomizer can be reduced.

### BRIEF DESCRIPTION OF THE DRAWINGS

One or more embodiments are exemplarily described with reference to the corresponding figures in the accompanying drawings, and the descriptions are not to be construed as limiting embodiments. Elements in the accompanying drawings that have same reference numerals are represented as similar elements, and unless otherwise particularly stated, the figures in the accompanying drawings are not drawn to scale.
FIG. 1 is a schematic diagram of a structure of an ultrasonic atomizer according to an embodiment of this application;
FIG. 2 is a schematic diagram of a structure of an ultrasonic atomizer according to another embodiment of this application;
FIG. 3 is a schematic diagram of a structure of a control branch according to an embodiment of this application;
FIG. 4 is a schematic diagram of a structure of a control branch according to another embodiment of this application;
FIG. 5 is a schematic diagram of a circuit structure of a control branch according to an embodiment of this application;
FIG. 6 is a flowchart of a method for determining a working state of an ultrasonic atomizer according to an embodiment of this application;
FIG. 7 is a schematic diagram of a structure of an apparatus for determining a working state of an ultrasonic atomizer according to an embodiment of this application; and
FIG. 8 is a schematic diagram of a structure of a controller according to an embodiment of this application.

### DETAILED DESCRIPTION

To make objectives, technical solutions, and advantages of embodiments of this application clearer, the technical solutions in embodiments of this application are clearly and completely described below with reference to the accompanying drawings in embodiments of this application. Apparently, the described embodiments are some rather than all of embodiments of this application. All other embodiments obtained by a person of ordinary skill in the art based on embodiments of this application without making creative efforts shall fall within the protection scope of this application.

According to an ultrasonic atomizer in an embodiment of this application, the ultrasonic atomizer stores, by using an energy storage branch, energy for a first drive signal driving an ultrasonic atomization sheet, and outputs a first detection voltage to a controller, so that the controller determines a current working state of the ultrasonic atomizer based on the first detection voltage. Regardless of whether the first drive signal is a high frequency signal or a low frequency signal, the energy storage branch can store energy and finally obtain the first detection voltage. In other words, a variety of different first drive signals can be used, showing strong practicality. In addition, for the ultrasonic atomizer, there is a large difference between a voltage when the ultrasonic atomizer is in abnormal such as dry burning and a voltage when the ultrasonic atomizer works normally. Therefore, obtaining the first detection voltage can accurately determine whether the ultrasonic atomizer is in abnormal, so that processing can be performed timely when the ultrasonic atomizer is in abnormal. In this way, a probability that the ultrasonic atomizer is damaged due to an abnormal condition such as dry burning can be reduced, and a risk of damage to the ultrasonic atomizer is low.

In this embodiment, the high frequency signal is usually a signal with a frequency greater than or equal to 1 MHz, and the low frequency signal is usually a signal with a frequency less than 1 MHz.

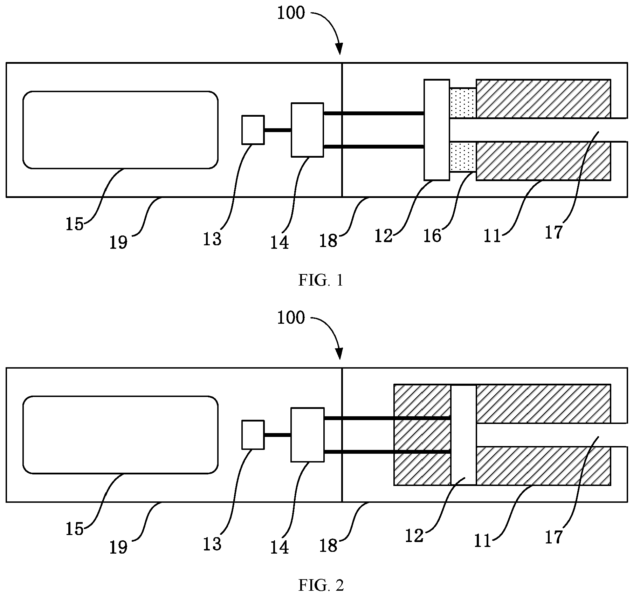

FIG. 1 is a schematic diagram of a structure of an ultrasonic atomizer according to an embodiment of this application. As shown in FIG. 1, the ultrasonic atomizer 100 includes a liquid storage cavity 11, an ultrasonic atomization sheet 12, a controller 13, a control circuit 14, and a power supply 15.

The liquid storage cavity 11 is configured to store a liquid substrate. The liquid substrate may include different substances based on different usage scenarios. For example, in the field of electronic cigarette atomization, the liquid substrate may include nicotine and/or aromatics and/or an aerosol-generation substance (for example, glycerin). For example, in the field of medical atomization, the liquid substrate may include solvents such as drugs and/or physiological saline that treat diseases or are beneficial to health.

The ultrasonic atomization sheet 12 being in fluid communication with the liquid storage cavity 11 may be that the ultrasonic atomization sheet 12 is directly disposed in the liquid storage cavity 11, or an atomization cavity in which the ultrasonic atomization sheet 12 is located is directly communicated with the liquid storage cavity 11, or liquid transfer is performed between the ultrasonic atomization sheet 12 and the liquid storage cavity 11 via a liquid absorbing medium. The ultrasonic atomization sheet 12 is configured to generate oscillation to atomize the liquid substrate. To be specific, the liquid substrate transferred to or near the ultrasonic atomization sheet 12 is atomized into an aerosol through oscillation. Specifically, during use, the ultrasonic atomization sheet 12 disperses the liquid substrate through high frequency oscillation (preferably an oscillation frequency is 1.7 MHz to 4.0 MHz, which exceeds a human hearing range and is within an ultrasonic frequency band) to generate an aerosol in which particles are naturally suspended.

The controller 13 may be a micro controller unit (MCU), a digital signal processing (DSP) controller, or the like. The controller 13 is electrically connected to the control circuit 14. The controller 13 may be configured to control at least one electronic element in the control circuit 14. The control circuit 14 is electrically connected to the ultrasonic atomization sheet 12. The control circuit 14 is configured to provide a drive voltage and a drive current to the ultrasonic atomization sheet 12 by the power supply 15. In an implementation, the controller 13 and the control circuit 14 may be disposed on a printed circuit board (PCB).

The power supply 15 is configured to supply power. In an implementation, the power supply 15 is a battery. The battery may be a lithium-ion battery, a lithium metal battery, a lead-acid battery, a nickel-cadmium battery, a nickel-metal hydride battery, a lithium-sulfur battery, a lithium-air battery, a sodium-ion battery, or the like. This is not limited herein. In terms of scale, the battery in this embodiment of this application may be a battery cell, a battery module including a plurality of battery cells connected in series and/or in parallel, or the like. This is not limited herein. Certainly, in another embodiment, the battery may alternatively include more or fewer elements, or have different element configurations. This is not limited in embodiments of this application.

In an embodiment, the ultrasonic atomizer 100 further includes a liquid transfer medium 16, an air outlet channel 17, an upper housing 18, and a lower housing 19.

The liquid transfer element 16 is configured to transfer the liquid substrate between the liquid storage cavity 11 and the ultrasonic atomization sheet 12.

The air outlet channel 17 is configured to output inhalable vapor or an aerosol generated by the liquid substrate for a user to inhale.

The upper housing 18 is detachably connected to the lower housing 19. In an embodiment, the upper housing 18 may be detachably connected to the lower housing 19 by using a snap structure, a magnetic structure, or the like. The upper housing 18 and the lower housing 19 jointly serve to accommodate and protect other elements and components. The liquid storage cavity 11, the ultrasonic atomization sheet 12, the liquid transfer element 16, and the air outlet channel 17 are all provided in the upper housing 18, and the controller 13, the control circuit 14, and the power supply 15 are all disposed in the lower housing 19.

The upper housing 18 is detachably aligned with the lower housing 19 in a functional relationship. Various mechanisms may be used to connect the lower housing 19 to the upper housing 18, to result in a threaded engagement, a press-fit engagement, an interference fit, a magnetic engagement, or the like. In some implementations, when the upper housing 18 and the lower housing 19 are in an assembled configuration, the ultrasonic atomizer 100 may be substantially in a shape of a stick, a flat cylinder, a rod, a column, or the like.

The upper housing 18 and lower housing 19 may be formed of any suitable material with an intact structure. In some examples, the upper housing 18 and the lower housing 19 may be formed of a metal or an alloy such as stainless steel or aluminum. Another suitable material includes various plastics (for example, polycarbonate), metal-plating over plastic (metal-plating over plastic), a ceramic, and the like.

It should be noted that a hardware structure of the ultrasonic atomizer 100 shown in FIG. 1 is merely an example, and the ultrasonic atomizer 100 may have more or fewer components than those shown in the figure, may combine two or more components, or may have different component configurations. The components shown in the figures may be implemented by hardware, software, or a combination of hardware and software including one or more signal processing and/or application-specific integrated circuits. For example, as shown in FIG. 2, the ultrasonic atomization sheet 12 may be disposed in the liquid storage cavity 11, so that the liquid transfer element 16 can be omitted, thereby reducing costs.

In addition, it may be understood that the ultrasonic atomizer 100 shown in FIG. 1 or FIG. 2 may be used in a variety of different situations and have different functions. This is not specifically limited in embodiments of this application. For example, in an embodiment, the ultrasonic atomizer 100 is used in the medical field. In this case, the ultrasonic atomizer 100 may be a medical atomizer. The medical atomizer can atomize medicinal liquid added inside the medical atomizer, and allow a patient to inhale to achieve an effect of auxiliary treatment. For another example, in another embodiment, the ultrasonic atomizer 100 may alternatively be used as an electronic product, such as an electronic cigarette. The electronic cigarette is an electronic product that converts nicotine solution and the like into an aerosol by using a means such as atomization for a user to inhale.

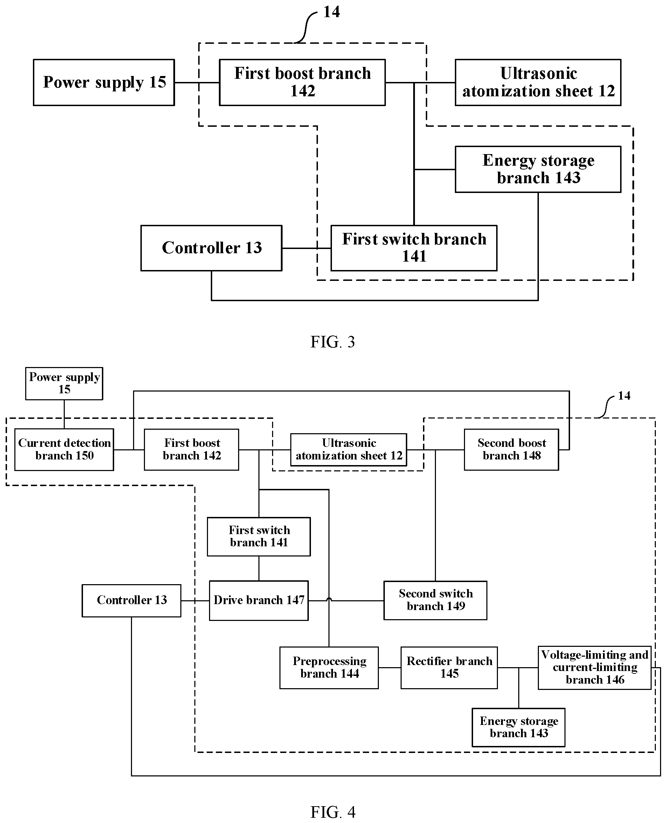

FIG. 3 is a schematic diagram of a circuit structure of an ultrasonic atomizer according to an embodiment of this application. As shown in FIG. 3, the control circuit 14 includes a first switch branch 141, a first boost branch 142, and an energy storage branch 143. The first switch branch 141 is connected to the controller 13. The first boost branch 142 is separately connected to the power supply 15, the first switch branch 141, and the ultrasonic atomization sheet 12. The energy storage branch 143 is separately connected to the first boost branch 142, the ultrasonic atomization sheet 12, and the controller 13.

Specifically, a first end of the first boost branch 142 is connected to the power supply 15, and a second end of the first boost branch 142 is separately connected to a first end of the energy storage branch 143, a first end of the first switch branch 141, and the ultrasonic atomization sheet 12. A second end of the energy storage branch 143 is connected to the controller 13. A second end of the first switch branch 141 is connected to the controller 13.

In this embodiment, the first switch branch 141 is configured to be turned on or turned off in response to a first pulse signal outputted by the controller 13. The first boost branch 142 is configured to boost an output voltage of the power supply 15 in response to the turning-on or the turning-off of the first switch branch 141, to output a first drive signal for driving the ultrasonic atomization sheet 12. The energy storage branch 143 is configured to: store energy in response to the first drive signal, and output a first detection voltage to the controller 13, so that the controller 13 determines a current working state of the ultrasonic atomizer 100 based on the first detection voltage. The current working state of the ultrasonic atomizer 100 includes a normal working state and an abnormal working state. The abnormal working state includes a state such as dry burning of the ultrasonic atomizer 100 that may cause damage to the ultrasonic atomizer 100.

Specifically, in a process of driving the ultrasonic atomization sheet 12 by the first drive signal, in other words, in a working process of the ultrasonic atomization sheet 12, the energy storage branch 143 simultaneously stores energy in response to the first pulse signal, until a voltage of the energy storage branch 143 no longer changes, and the voltage of the energy storage branch 143 is the first detection voltage. After receiving the first detection voltage, the controller 13 may determine the current working state of the ultrasonic atomizer 100 based on a magnitude the first detection voltage.

It may be understood that for the ultrasonic atomizer 12, there is a large difference between a voltage of the first drive signal when the ultrasonic atomizer 12 is in abnormal such as dry burning and a voltage of the first drive signal when the ultrasonic atomizer 12 works normally. In this case, whether the current working state of the ultrasonic atomizer 100 is the normal working state or the abnormal working state may be determined more accurately based on the first detection voltage. In this way, when the ultrasonic atomizer 100 is in abnormal, processing can be performed timely, to reduce a risk of damage to the ultrasonic atomizer 100.

In addition, in this embodiment, both the high frequency signal and the low frequency signal can implement an energy storage process of the energy storage branch 143 and generate the first detection voltage to determine the current working state of the ultrasonic atomizer 100. Therefore, the first drive signal may be a high frequency signal or a low frequency signal. In other words, in application scenarios in which different ultrasonic atomization sheets 12 are used, first drive signals with different frequencies need to be used, and the energy storage branch 143 can be used in these application scenarios to obtain the first detection voltage, showing strong practicality.

In an embodiment, as shown in FIG. 4, the control circuit 14 further includes a preprocessing branch 144, a rectifier branch 145, and a voltage-limiting and current-limiting branch 146. The preprocessing branch 144 is separately connected to the first boost branch 142, the first switch branch 141, and the ultrasonic atomization sheet 12. The rectifier branch 145 is connected to the preprocessing branch 144. The voltage-limiting and current-limiting branch 146 is separately connected to the rectifier branch 145, the energy storage branch 143, and the controller 13.

Specifically, a first end of the preprocessing branch 144 is connected to the first end of the first switch branch 141, and a second end of the preprocessing branch 144 is connected to a first end of the rectifier branch 145. A second end of the rectifier branch 145 is separately connected to the first end of the energy storage branch 143 and a first end of the voltage-limiting and current-limiting branch 146. A second end of the voltage-limiting and current-limiting branch 146 is connected to the controller 13.

The preprocessing branch 144 is configured to: perform direct current blocking, voltage division, and filtering on the first drive signal, and output a first drive sub-signal. The rectifier branch 145 is configured to rectify the first drive sub-signal, so that the energy storage branch 143 stores energy in response to the first drive sub-signal and outputs the first detection voltage. The voltage-limiting and current-limiting branch 146 is configured to: perform voltage limiting and current limiting on the first detection voltage, and output a second detection voltage to the controller 13, so that the controller 13 determines the current working state of the ultrasonic atomizer 100 based on the second detection voltage.

In this embodiment, in the working process of the ultrasonic atomization sheet 12, to determine the voltage of the first drive signal, first, the preprocessing branch 144 is used to perform direct current blocking, voltage division, and filtering on the first drive signal. Through the direct current blocking, a possible direct current may be blocked to prevent the direct current from interfering with a detection signal inputted to the controller 13, causing the controller 13 to obtain a wrong detection signal, and further leading to misjudgment. In other words, through the direct current blocking, a probability of misjudgment can be reduced. Through the voltage division, an amplitude of the first drive signal can be reduced, to reduce a voltage inputted to the controller 13, so as to protect the controller 13. Through the filtering, a possible high-voltage pulse signal is filtered out to protect a subsequent electronic element, for example, the controller 13.

Then, the preprocessing branch 144 outputs the first drive sub-signal, and the first drive sub-signal is rectified by the rectifier branch 143 to rectify the first drive sub-signal into a signal capable of charging the energy storage branch 143.

Further, the energy storage branch 143 stores energy based on a rectified first drive sub-signal, and the voltage of the energy storage branch 143 gradually increases until the voltage of the energy storage branch 143 keeps in a stable state. For example, when the voltage of the energy storage branch 143 is within a preset voltage range, it may be considered that the voltage of the energy storage branch 143 keeps in a stable state. In this case, the voltage of the energy storage branch 143 is denoted as the first detection voltage, and the first detection voltage is transmitted to the voltage-limiting and current-limiting branch 146. Even though the first drive sub-signal is a rapidly changing value (that is, a signal with a high frequency), the energy storage branch 143 can perform multiple accumulations based on the first drive sub-signal to obtain a variable that can reflect an amplitude and a frequency of the first drive sub-signal, and input the variable to the controller 13. It can be learned that the signal received by the controller 13 is not a rapidly changing value, but a variable obtained through accumulation, so that a processing requirement for the controller 13 is not high. In other words, even though a controller 13 with a low sampling frequency is selected, a sampling requirement for the first detection voltage can be satisfied. As the sampling frequency decreases, the price of the controller 13 also decreases. In this case, the controller 13 with a low sampling frequency is selected to reduce costs.

After receiving the first detection voltage, the voltage-limiting and current-limiting branch 146 performs voltage limiting of the first detection voltage to prevent the voltage from rising excessively, and performs current limiting on the first detection voltage to prevent an excessive current from flowing into the controller 13, so as to protect the controller 13. In addition, the voltage-limiting and current-limiting branch 146 further outputs the second detection voltage to the controller 13.

After receiving the second detection voltage, the controller 13 may determine the current working state of the ultrasonic atomizer 100 based on the second detection voltage.

Specifically, in an optional implementation, after receiving the second detection voltage, if it is determined that the second detection voltage is greater than or equal to a second voltage threshold, the controller 13 determines that the current working state of the ultrasonic atomizer 100 is the abnormal working state. The second voltage threshold may be set based on an actual selected frequency of the ultrasonic atomization sheet 12. This is not specifically limited in embodiments of this application.

In an embodiment, reference is still made to FIG. 4. The control circuit 14 further includes a drive branch 147. The drive branch 147 is separately connected to the controller 13, the first switch branch 141, and the power supply 15.

Specifically, the drive branch 147 is configured to output a second pulse signal in response to a current outputted by the power supply 15 and the first pulse signal. To be specific, the drive branch 147 is configured to enhance a drive capability of the first pulse signal and output a second pulse signal to satisfy a requirement for quick switching on and off of the first switch branch 141, so as to provide a more stable first drive signal to the ultrasonic atomization sheet 12 to maintain stable working of the ultrasonic atomization sheet 12. A drive capability of the second pulse signal is stronger than that of the first pulse signal.

In an embodiment, reference is still made to FIG. 4. The control circuit 14 further includes a second boost branch 148 and a second switch branch 149. The second boost branch 148 is separately connected to the power supply 15, the second switch branch 149, and the ultrasonic atomization sheet 12. The second switch branch 149 is connected to the drive branch 147.

Specifically, the second switch branch 149 is configured to be turned on or turned off in response to the second pulse signal. The second boost branch 148 is configured to boost an output voltage of the power supply 15 in response to the turning-on or the turning-off of the second switch branch 149, to output a second drive signal for driving the ultrasonic atomization sheet 12. The drive branch 147 is further configured to output a fourth pulse signal in response to the current outputted by the power supply 15 and a third pulse signal outputted by the controller 13.

In an embodiment, reference is still made to FIG. 4. The control circuit 14 further includes a current detection branch 150. The current detection branch 150 is separately connected to the power supply 15, the first boost branch 142, and the controller 13.

Specifically, the current detection branch 150 is configured to detect a current flowing into the first boost branch 142.

In this embodiment, the controller 13 may obtain the current flowing into the first boost branch 142 by using the current detection branch 150. Further, the controller 13 may determine, based on the current, whether an abnormal condition such as an excessive current occurs in the working process of the ultrasonic atomization sheet 12, so that processing can be performed timely when there is an abnormal condition, thereby reducing a risk of damage to the ultrasonic atomization sheet 12.

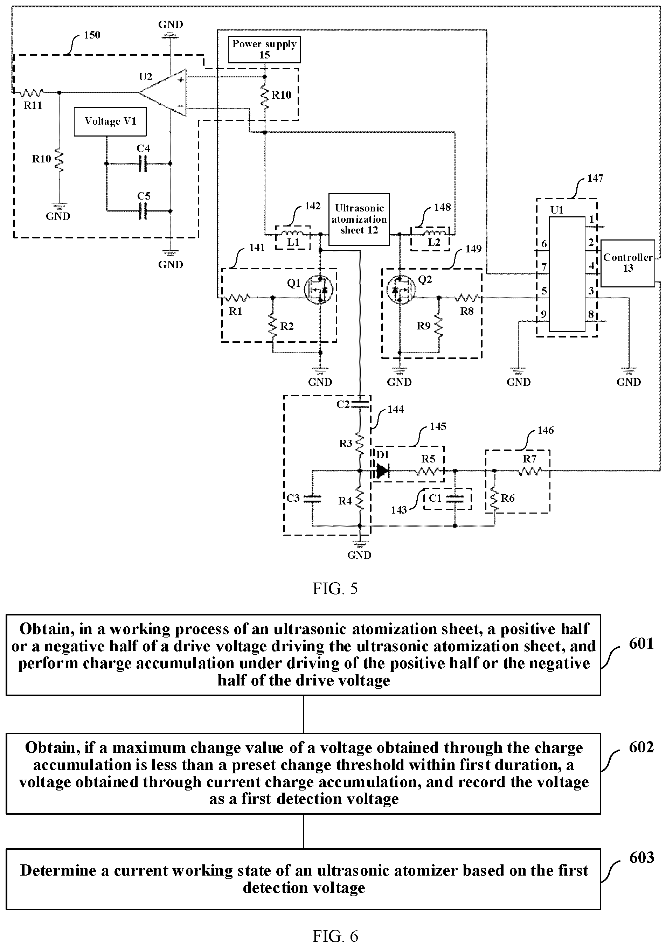

FIG. 5 exemplarily shows a circuit structure corresponding to the structure of the ultrasonic atomizer 100 shown in FIG. 4.

As shown in FIG. 5, in an embodiment, the drive branch 147 includes a drive chip U1, and the drive chip U1 includes a power supply input end, a first signal input end, a second signal input end, a first signal output end, and a second signal output end. In this embodiment, the power supply input end is a sixth pin of the drive chip U1; the first signal input end is a second pin of the drive chip U1; the second signal input end is a fourth pin of the drive chip U1; the first signal output end is a seventh pin of the drive chip U1; and the second signal output end is a fifth pin of the drive chip U1.

Specifically, the sixth pin of the drive chip U1 is configured to be connected to the power supply 15. Both the second pin and the fourth pin of the drive chip U1 are connected to the controller. The fifth pin of the drive chip U1 is connected to the second switch branch 149, and the seventh pin of the drive chip U1 is connected to the first switch branch 141. The second pin of the drive chip U1 is configured to input a first pulse signal; the fourth pin of the drive chip U1 is configured to input a second pulse signal; the seventh pin of the drive chip U1 is configured to output a third pulse signal; and the fifth pin of the drive chip U1 is configured to output a fourth pulse signal.

In this embodiment, the drive chip U1 is provided to enhance a drive capability of a pulse signal outputted by the controller 13. In this way, the first switch branch 141 and the second switch branch 149 can be driven quickly to maintain stable operation of the ultrasonic atomization sheet 13. In addition, a greater current inputted by the sixth pin of the drive chip U1 indicates stronger drive capabilities outputted by the fifth pin and the seventh pin of the drive chip U1.

In an embodiment, an integrated chip of a model SGM48000 may be used as the drive chip U1. Certainly, in another embodiment, another type of integrated chip may alternatively be selected. This is not limited in embodiments of this application. In addition, because there are different types of drive chips, when another type of drive chip is used, definitions of specific pins may be different, but functions and signal definitions of the specific pins are the same. If another type of drive chip is selected, a manner similar to the foregoing embodiment may be used. This is within the scope that is easily understood by a person of skill in the art. Details are not described herein again.

In addition, in this embodiment, an example in which the power supply 15 is used as an input power supply of the drive chip U1 is used. In other words, in this embodiment, the power supply 15 is used as a power supply for both the drive chip U1 and the ultrasonic atomization sheet 12 to reduce costs. However, in another embodiment, to prevent the drive chip U1 and the ultrasonic atomization sheet 12 from interfering with each other during working, two different power supplies may be used to respectively supply power to the drive chip U1 and the ultrasonic atomization sheet 12 to improve working stability of the drive chip U1 and the ultrasonic atomization sheet 12.

In an embodiment, the first switch branch 141 includes a first switch Q1. A first end of first switch Q1 is connected to controller 13 by the drive branch 147, and the first end of the first switch Q1 is connected to the seventh pin of the drive chip U1. A second end of the first switch Q1 is connected to the ground GND. A third end of the first switch Q1 is separately connected to the first boost branch 142 and the ultrasonic atomization sheet 12.

In this embodiment, an example in which the first switch Q1 is an N-type metal-oxide-semiconductor field-effect transistor (referred to as an NMOS transistor below) is used. A gate of the NMOS transistor is the first end of the first switch Q 1; a source of the NMOS transistor is the second end of the first switch Q1; and a drain of the NMOS transistor is the third end of the first switch Q1.

In addition, in other embodiments, the first switch Q1 may alternatively be a P-type metal-oxide-semiconductor field-effect transistor or a signal relay. The first switch Q1 may alternatively be at least one of a triode, an insulated gate bipolar transistor, an integrated gate-commutated thyristor, a gate turn-off thyristor, a junction gate field-effect transistor, an MOS controlled thyristor, a gallium-nitride-based power device, a silicon-carbide-based power device, or a silicon controlled rectifier.

In an embodiment, the first switch branch 141 further includes a first resistor R1 and a second resistor R2. A first end of the first resistor R1 is connected to the controller 13 by the drive branch 147. A second end of the first resistor R1 is separately connected to the first end of the first switch Q1 and a first end of the second resistor R2. A second end of the second resistor R2 is connected to the ground GND.

In this embodiment, the first resistor R1 and the second resistor R2 are configured to perform voltage division on a voltage of a pulse signal outputted by the drive branch 147 to obtain a voltage at the first end of the first switch Q1. When a divided voltage on the second resistor R2 is higher than a turn-on voltage of the first switch Q1, the first switch Q1 is turned on. Otherwise, the first switch Q1 is turned off.

In an embodiment, the second switch branch 149 includes a second switch Q2, an eighth resistor R8, and a ninth resistor R9. A first end of the second switch Q2 is connected to the fifth pin of the drive chip U1 in the drive branch 147, and a third end of the second switch Q2 is separately connected to the ultrasonic atomization sheet 12 and the second boost branch 148. A first end of the eighth resistor R8 is connected to the controller 13 by the drive branch 147. In other words, the first end of the eighth resistor R8 is connected to the fifth pin of the drive chip U1. A second end of the eighth resistor R8 is connected to a first end of the ninth resistor R9. Both a second end of the second switch Q2 and a second end of the ninth resistor R9 are connected to the ground GND.

In this embodiment, an example in which the second switch Q2 is an N-type metal-oxide-semiconductor field-effect transistor (referred to as an NMOS transistor below) is used. A gate of the NMOS transistor is the first end of the second switch Q2; a source of the NMOS transistor is the second end of the second switch Q2; and a drain of the NMOS transistor is the third end of the second switch Q2.

In addition, in other embodiments, the second switch Q2 may alternatively be a P-type metal-oxide-semiconductor field-effect transistor or a signal relay. The second switch Q2 may alternatively be at least one of a triode, an insulated gate bipolar transistor, an integrated gate-commutated thyristor, a gate turn-off thyristor, or a junction gate field-effect transistor, an MOS controlled thyristor, a gallium-nitride-based power device, a silicon-carbide-based power device, or a silicon controlled rectifier.

The eighth resistor R8 and the ninth resistor R9 are configured to perform voltage division on a voltage of a pulse signal outputted by the drive branch 147 to obtain a voltage at the first end of the second switch Q2. When a divided voltage on the ninth resistor R9 is higher than a turn-on voltage of the second switch Q2, the second switch Q2 is turned on. Otherwise, the second switch Q2 is turned off.

In this embodiment, when the first switch Q1 is turned on and the second switch Q2 is turned off, the power supply 15, a first inductor L1, and the first switch Q1 form a loop, and the first inductor L1 is charged by the power supply 15. In addition, the power supply 15, a second inductor L2, the ultrasonic atomization sheet 12, and the first switch Q1 form a loop, and voltages on the power supply 15 and the second inductor L2 simultaneously provide a drive voltage for the ultrasonic atomization sheet 12.

When the second switch Q2 is turned on and the first switch Q1 is turned off, the power supply 15, the second inductor L2, and the second switch Q2 form a loop, and the second inductor L2 is charged by the power supply 15. In addition, the power supply 15, the first inductor L1, the ultrasonic atomization sheet 12, and the second switch Q2 form a loop, and voltages on the power supply 15 and the first inductor L1 simultaneously provide a drive voltage for the ultrasonic atomization sheet 12.

In an embodiment, the first boost branch 142 includes the first inductor L1. A first end of the first inductor L1 is connected to the power supply 15, and a second end of the first inductor L1 is connected to the third end of the first switch Q1 in the first switch branch 141 and the ultrasonic atomization sheet 12.

Specifically, the first inductor L1 is configured to be charged when the first switch Q1 is turned on, and generate, based on a voltage of the power supply 15 and a voltage charged by the first inductor L 1 when the first switch Q 1 is turned off, a first drive signal for driving the ultrasonic atomization sheet 12.

In an embodiment, the second boost branch 148 includes the second inductor L2. A first end of the second inductor L2 is connected to the power supply 15, and a second end of the second inductor L2 is separately connected to the third end of the second switch Q2 in the second switch branch 149 and the ultrasonic atomization sheet 12.

Specifically, the second inductor L2 is configured to be charged when the second switch Q2 is turned on, and generate, based on a voltage of the power supply 15 and a voltage charged by the second inductor L2 when the second switch Q2 is turned off, a first drive signal for driving the ultrasonic atomization sheet 12.

In an embodiment, the preprocessing branch 144 includes a second capacitor C2, a third capacitor C3, a third resistor R3, and a fourth resistor R4 A first end of the second capacitor C2 is separately connected to the second end of the first inductor L1 in the first boost branch 142, the third end of the first switch Q1 in the first switch branch 141, and the ultrasonic atomization sheet 12. A second end of the second capacitor C2 is connected to a first end of the third resistor R3. A second end of the third resistor R3 is separately connected to a first end of the third capacitor C3, a first end of the fourth resistor R4, and the rectifier branch 145. A second end of the third capacitor C3 and a second end of the fourth resistor R4 are connected to the ground GND.

In this embodiment, the second capacitor C2 is configured to perform direct current blocking. A combination of the third resistor R3 and the fourth resistor R4 is configured to divide a voltage. The third capacitor C3 is configured to perform filtering.

In an embodiment, the rectifier branch 145 includes a first diode D1 and a fifth resistor R5. An anode of the first diode D1 is connected to a connection point between the third resistor R3 and the fourth resistor R4 in the preprocessing branch 144, and a cathode of the first diode D1 is connected to a first end of the fifth resistor R5. A second end of the fifth resistor R5 is separately connected to the energy storage branch 143 and the voltage-limiting and current-limiting branch 146.

In this embodiment, due to unidirectional conductivity of the first diode D1, the first diode D1 only allows a signal greater than 0. This is equivalent to filtering out a negative half of a first drive sub-signal outputted by the preprocessing branch 144, leaving only a positive half. In addition, the first diode D1 can further effectively prevent a voltage of a circuit connected to the cathode of the first diode D1 from flowing back to a circuit connected to the anode of the first diode D1, to protect the circuit (for example, the ultrasonic atomization sheet 12) connected to the anode of the first diode D 1.

In an embodiment, the energy storage branch 143 includes a first capacitor C1. After passing the rectifier branch 145 and the preprocessing branch 144, a first end of the first capacitor C1 is separately connected to the second end of the first inductor L1 in the first boost branch 142, the third end of the first switch Q1 in the first switch branch 141, and the ultrasonic atomization sheet 12. A second end of the first capacitor C1 is connected to the ground GND.

Specifically, the first drive sub-signal outputted by the rectifier branch 145 can charge the first capacitor C1. When a voltage across two ends of the first capacitor C1 is a stable voltage, for example, the voltage across the two ends of the first capacitor C1 are within a preset voltage range, the voltage across the two ends of the first capacitor C1 is denoted as a first detection voltage. It may be learned that the first capacitor C1 can accumulate charges under driving of the first drive sub-signal to obtain a variable that can reflect an amplitude and a frequency of the first drive sub-signal, and input the variable to the controller 13. Therefore, the signal received by the controller 13 is not a rapidly changing value, but a variable obtained through accumulation. In other words, a processing requirement for the controller 13 is not high. Even though a controller 13 with a low sampling frequency is selected, a sampling requirement for the first detection voltage can be satisfied. In this case, the controller 13 with a low sampling frequency can be selected to reduce costs.

In an implementation, a capacitance value of the first capacitor C 1 is less than or equal to 100 nF. Selecting the capacitance value of the first capacitor C1 that is less than or equal to 100 nF can reduce a risk of damage to the first capacitor C1 caused by breakdown while generating a stable first detection voltage at a fast speed, thereby improving working stability of the ultrasonic atomizer 100.

In an embodiment, the voltage-limiting and current-limiting branch 146 includes a sixth resistor R6 and a seventh resistor R7. A first end of the sixth resistor R6 is separately connected to a first end of the seventh resistor R7, the rectifier branch 145, and the energy storage branch 143, and a second end of the sixth resistor R6 is connected to the ground GND. A second end of the seventh resistor R7 is connected to the controller 13.

In this embodiment, the sixth resistor R6 is configured to provide a small load to perform voltage limiting on the first detection voltage, so as to prevent the voltage from rising excessively. The seventh resistor R7 is configured to perform current limiting on the first detection voltage to prevent an excessive current from flowing into the controller 13, so as to protect the controller 13.

In an embodiment, the current detection branch 150 includes an amplifier U2 and a tenth resistor R10. The tenth resistor R10 is separately connected to the amplifier U2 and the first boost branch 142, and the amplifier U2 is connected to the controller 13.

Specifically, a first end of the tenth resistor R10 is separately connected to the power supply 15 and a non-inverting input end of the amplifier U2, and a second end of the tenth resistor R10 is separately connected to an inverting input end of the amplifier U2, the first end of the first inductor L1, and the first end of the second inductor L2. An output end of the amplifier U2 is connected to the controller 13. A ground end of the amplifier U2 is connected to the ground GND. A power supply end of the amplifier U2 is connected to a voltage V1.

In this embodiment, the amplifier U2 is configured to output a detection voltage based on a voltage across two ends of the tenth resistor R10, so that the controller 13 determines, based on the detection voltage, a current flowing into first boost branch 142 or the second boost branch 148. Specifically, the amplifier U2 can amplify the received voltage across the two ends of the tenth resistor R10 by K times and then output the detection voltage. K is a positive integer. Further, after obtaining the detection voltage, the controller 13 may determine, based on a relationship between the detection voltage and a current flowing into the first boost branch 142 or the second boost branch 148, the current flowing into the first boost branch 142 or the second boost branch 148.

In an embodiment, the current detection branch 144 includes a fourth capacitor C4, a fifth capacitor C5, a tenth resistor R10, and an eleventh resistor R11. The fourth capacitor C4 and the fifth capacitor C5 are filter capacitors, the tenth resistor R10 is a pull-down resistor, and the eleventh resistor R11 is a current limiting resistor.

It should be noted that, in embodiments shown in the foregoing figures, the resistor is represented as a single resistor, and the capacitor is represented as a single capacitor. In other embodiments, the resistor may alternatively be an integration of resistors that are connected in series, connected in parallel, or connected in series and parallel, and the capacitor may alternatively be an integration of capacitors that are connected in series, connected in parallel, or connected in series and parallel.

The connection described in this application may be a direct connection, to be specific, a connection between two devices, or an indirect connection, to be specific, an indirect connection between two components by one or more elements.

FIG. 6 is a schematic flowchart of a method for determining a working state of an ultrasonic atomizer according to an embodiment of the present invention. The method may be performed by the ultrasonic atomizer shown in FIG. 1 to FIG. 5. For a structure of the ultrasonic atomizer, reference may be made to the foregoing detailed descriptions of FIG. 1 to FIG. 5. Details are not described herein again. As shown in FIG. 6, the method for determining a working state of an ultrasonic atomizer includes the following steps.

Step 601: Obtain, in a working process of the ultrasonic atomization sheet, a positive half or a negative half of a drive voltage driving the ultrasonic atomization sheet, and perform charge accumulation under driving of the positive half or the negative half of the drive voltage.

In this embodiment, the drive voltage for driving the ultrasonic atomization sheet is an alternating-current signal. If the alternating-current signal is directly used, the charge accumulation may not be performed due to a positive half and a negative half of the alternating-current signal. In this case, to perform the charge accumulation, only the positive half of the drive voltage or only the negative half of the drive voltage is retained. In an implementation, a diode may be provided to filter out the negative half of the drive voltage to retain the positive half.

Step 602: Obtain, if a maximum change value of a voltage obtained through the charge accumulation is less than a preset change threshold within first duration, a voltage obtained through current charge accumulation, and record the voltage as a first detection voltage.

Charges continue to accumulate under driving of the drive voltage. When the maximum change value (that is, a difference between a maximum value and a minimum value within the first duration) of the voltage obtained through the charge accumulation keeps less than the preset change threshold within the first duration, a voltage obtained through the charge accumulation at this time is recorded as the first detection voltage. In this case, it may be considered that the charges keep in a stable state. In other words, the first detection voltage is a stable signal.

The preset change threshold may be set based on actual application. This is not specifically limited in embodiments of this application. For example, in an embodiment, the preset change threshold may be set to 0.2 v. In this case, if the voltage obtained through the charge accumulation keeps within a range of [1 v, 1.1 v], and duration is longer than or equal to the first duration, the maximum change value of the voltage obtained through the charge accumulation is 1.1-1=0.1 v, which is less than 0.2 v. In this case, the voltage obtained through the charge accumulation may be considered as a stable voltage, and the first detection voltage may be obtained.

The first duration may be set based on actual application. This is not specifically limited in embodiments of this application. For example, in an implementation, the first duration is set to any duration in (0, 10 ms], for example, 10 ms. If the maximum change value of the voltage obtained through the charge accumulation can keep less than the preset change threshold within 10 ms, a fluctuation amplitude of the voltage obtained through the charge accumulation within 10 ms is small. In this case, the voltage obtained through the charge accumulation may be considered as a stable voltage, and the currently obtained voltage is recorded as the first detection voltage. Setting the first duration can accurately determine that the first detection voltage is a stable voltage, thereby reducing a probability of misjudgment.

For another example, in another implementation, the first duration is set to duration longer than or equal to five sampling periods, and each sampling period is any one of duration in (0, 100 µs]. The sampling period is a period in which the voltage obtained through the charge accumulation is sampled each time. If the voltage obtained through the charge accumulation are continuously sampled for five times or more, and the maximum change value of each sampled voltage obtained through the charge accumulation is less than the preset change threshold, it may be determined that the first detection voltage is a stable voltage.

For example, in an implementation, the preset change threshold is set to 0.2 v; the first duration is set to be equal to duration of five sampling periods; a voltage sampled for the first time is 0.4 v; a voltage sampled for the second time is 0.5 v; a voltage sampled for the third time is 0.5 v; a voltage sampled for the fourth time is 0.4 v; and a voltage sampled for the fifth time is 0.5 v. In this case, in this embodiment, the maximum change value of the voltage is 0.5-0.4=0.1 v<0.2 v. In this case, a current voltage (that is, 0.5 v) obtained through the charge accumulation may be used as the first detection voltage.

In this embodiment, in a manner of sampling a plurality of times, sampling errors caused by a voltage fluctuation due to environmental interference and the like can be avoided, and a probability of misjudgment can also be reduced. In addition, setting the sampling period to any one of duration in (0, 100 µs] can increase a probability of sampling the voltage.

Further, because there is a correspondence between the drive voltage and the first detection voltage, a magnitude of the drive voltage may be determined by obtaining the first detection voltage.

In addition, in this embodiment, regardless of whether the drive voltage is a rapidly changing value (that is, a signal with a high frequency) or a slowly changing value (that is, a signal with a low frequency), the charge accumulation can be implemented. Therefore, the method may be applied to signals with different frequencies. In other words, the method may be applied to various application scenarios, showing strong practicability. In addition, because the first detection voltage is a variable obtained through the charge accumulation, the variable has a low processing requirement on a controller. As the sampling frequency decreases, a price of the controller also decreases. In this case, a controller with a low sampling frequency may be selected to reduce costs when a sampling requirement for the first detection voltage is satisfied.

Step 603 : Determine a current working state of the ultrasonic atomizer based on the first detection voltage.

In this embodiment, the current working state of the ultrasonic atomizer is related to the drive voltage of the ultrasonic atomization sheet, and the drive voltage is related to the first detection voltage. Therefore, the first detection voltage is obtained to accordingly determine the current working state of the ultrasonic atomizer.

In an implementation, the determining a current working state of the ultrasonic atomizer based on the first detection voltage in step 603 specifically includes: determining, if the first detection voltage is less than a first voltage threshold, that the current state of the ultrasonic atomizer is a normal working state; or determining, if the first detection voltage is greater than or equal to a first voltage threshold, that the current working state of the ultrasonic atomizer is an abnormal working state.

The first voltage threshold may be set based on an actual selected frequency of the ultrasonic atomization sheet. This is not specifically limited in embodiments of this application. For example, in an implementation, an ultrasonic atomization sheet with a frequency of 3 MHz is selected, and the first voltage threshold may be set to any value in [0.9 v, 1 v], for example, 0.9 v. When the first detection voltage is greater than or equal to 0.9 v, it can be determined that the current working state of the ultrasonic atomizer is the abnormal working state. Therefore, when the ultrasonic atomizer 100 is in abnormal such as dry burning, the abnormal condition can be detected and dealt with timely, and an effect of protecting the electronic elements in the ultrasonic atomizer 100 is better. In addition, when the first detection voltage is greater than or equal to the first voltage threshold, the corresponding second detection voltage in the foregoing embodiment is greater than or equal to a second voltage threshold. A specific implementation process is described in detail in the foregoing embodiment. Details are not described herein again.

Furthermore, in an implementation, after it is determined that the current working state of the ultrasonic atomizer 100 is the abnormal working state, a user is prompt through an indicator light or an alarm provided by a buzzer, so that the user stops using the ultrasonic atomizer 100 timely, and accordingly deals with a specific abnormal condition. For example, if the abnormal working state of the ultrasonic atomizer 100 is dry burning, after the ultrasonic atomizer 100 is stopped using, a liquid substrate may be added to the ultrasonic atomizer 100 to prevent the dry burning from occurring again.

It should be understood that for specific control of the ultrasonic atomizer and beneficial effects generated in the method embodiment, reference may be made to corresponding descriptions in the foregoing apparatus embodiment. For brevity, details are not described herein again.

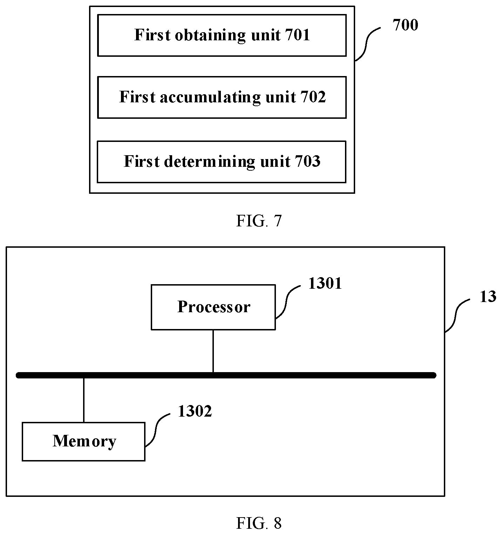

An embodiment of this application provides an apparatus for determining a working state of an ultrasonic atomizer. For a structure of the ultrasonic atomizer, reference may be made to the foregoing detailed descriptions of FIG. 1 to FIG. 5. Details are not described herein again. FIG. 7 is a schematic diagram of a structure of an apparatus for determining a working state of an ultrasonic atomizer according to an embodiment of this application. The apparatus 700 for determining a working state of an ultrasonic atomizer includes a first obtaining unit 701, a first accumulating unit 702, and a first determining unit 703.

The first obtaining unit 701 is configured to: obtain, in a working process of the ultrasonic atomization sheet, a positive half or a negative half of a drive voltage driving the ultrasonic atomization sheet, and perform charge accumulation under driving of the positive half or the negative half of the drive voltage.

The first accumulating unit 702 is configured to: obtain, if duration of a voltage obtained through the charge accumulation is longer than or equal to first duration, a voltage obtained by current charge accumulation, and record the voltage as a first detection voltage.

The first determining unit 703 is configured to determine a current working state of the ultrasonic atomizer based on the first detection voltage.

The foregoing product may perform the method provided in embodiments of this application shown in FIG. 6, and has corresponding functional modules for performing the method and beneficial effects of performing the method. For technical details that are not described in detail in this embodiment, reference may be made to the method provided in embodiments of this application.

FIG. 8 further exemplarily shows a structure of the controller 13. As shown in FIG. 8, the controller 13 includes at least one processor 1301 and a memory 1302 in communication connection with the at least one processor 1301. In FIG. 8, an example in which there is one processor 1301 is used.

The memory 1302 stores instructions executable by the at least one processor 1301, and the instructions are executed by the at least one processor 1301, to enable the at least one processor 1301 to perform the foregoing method for determining a working state of an ultrasonic atomizer in FIG. 6. The processor 1301 and the memory 1302 may be connected via a bus or in another manner. In FIG. 8, an example in which a bus is used for connection is used.

As a non-volatile computer-readable storage medium, the memory 1302 may be configured to store a non-volatile software program and a non-volatile computer-executable program and module, such as program instructions/units, for example, the units shown in FIG. 7, corresponding to the method for determining a working state of an ultrasonic atomizer in embodiments of this application. The processor 1301 runs the non-volatile software program, instructions, and module stored in the memory 1302 to perform various functional applications and data processing of a server, to be specific, to implement the method for determining a working state of an ultrasonic atomizer in the foregoing method embodiment.

The memory 1302 may include a program storage area and a data storage area. The program storage area may store an operating system and an application program that is required by at least one function. The data storage area may store data and the like created based on use of a data transmission apparatus. In addition, the memory 1302 may include a high-speed random access memory, and may alternatively include a non-volatile memory, for example, at least one magnetic disk storage device, a flash memory device, or another non-volatile solid-state storage device. In some embodiments, the memory 1302 may optionally include memories remotely disposed relative to the processor 1301, and the remote memories may be connected to the data transmission apparatus via a network. Examples of the network include but are not limited to the Internet, an intranet, a local area network, a mobile communication network, and a combination thereof.

One or more modules are stored in the memory 1302. When the one or more modules are executed by the one or more processors 1301, the method for determining a working state n any of the foregoing method embodiments is performed, for example, steps of the foregoing method in FIG. 6 are performed, to implement functions of the modules and the units in FIG. 7.

The foregoing product may perform the method provided in embodiments of this application, and has functional modules for performing the method and beneficial effects of performing the method. For technical details that are not described in detail in this embodiment, reference may be made to the method provided in embodiments of this application.

An embodiment of this application further provides a non-volatile computer-readable storage medium. The computer-readable storage medium stores computer-executable instructions. The computer-executable instructions are executed by one or more processors, for example, steps of the foregoing method in FIG. 6 are performed, to implement functions of the units in FIG. 7.

An embodiment of this application further provides a computer program product, including a computer program stored on a non-volatile computer-readable storage medium. The computer program includes program instructions. When the program instructions are executed by a computer, the computer is enabled to perform the method for determining a working state of an ultrasonic atomizer in any method embodiment, for example, to perform steps of the foregoing method in FIG. 6, to implement functions of the units in FIG. 7.

Finally, it should be noted that the foregoing embodiments are merely intended for describing the technical solutions of this application, but not for limiting this application. Under the ideas of this application, the technical features in the foregoing embodiments or different embodiments may also be combined, the steps may be performed in any order, and many other changes of different aspects of this application also exists as described above, and these changes are not provided in detail for simplicity. Although this application are described in detail with reference to the foregoing embodiments, a person of ordinary skill in the art should understand that modifications may still be made to the technical solutions described in the foregoing embodiments, or equivalent replacements may be made to some technical features, and these modifications or replacements do not cause the essence of corresponding technical solutions to depart from the scope of the technical solutions in embodiments of this application.

## Claims

1. An ultrasonic atomizer, comprising:
a liquid storage cavity, configured to store a liquid substrate;
an ultrasonic atomization sheet, configured to generate oscillation to atomize the liquid substrate; and
a controller, a control circuit, and a power supply, wherein:
the control circuit comprises:
a first switch branch, connected to the controller, wherein the first switch branch is configured to turn on or turn off in response to a first pulse signal outputted by the controller;
a first boost branch, separately connected to the power supply, the first switch branch, and the ultrasonic atomization sheet, wherein the first boost branch is configured to boost an output voltage of the power supply in response to the turning-on or the turning-off of the first switch branch, to output a first drive signal for driving the ultrasonic atomization sheet; and
an energy storage branch, separately connected to the first boost branch, the ultrasonic atomization sheet, and the controller, wherein the energy storage branch is configured to: store energy in response to the first drive signal, and output a first detection voltage to the controller, so that the controller determines a current working state of the ultrasonic atomizer based on the first detection voltage.

2. The ultrasonic atomizer according to claim 1, wherein:
the first switch branch comprises a first switch;
a first end of the first switch is connected to the controller;
a second end of the first switch is connected to the ground; and
a third end of the first switch is separately connected to the first boost branch and the ultrasonic atomization sheet.

3. The ultrasonic atomizer according to claim 2, wherein:
the first switch branch further comprises a first resistor and a second resistor;
a first end of the first resistor is connected to the controller;
a second end of the first resistor is separately connected to the first end of the first switch and a first end of the second resistor; and
a second end of the second resistor is connected to the ground.

4. The ultrasonic atomizer according to claim 1, wherein:
the first boost branch comprises a first inductor;
a first end of the first inductor is connected to the power supply; and
a second end of the first inductor is connected to the first switch branch and the ultrasonic atomization sheet.

5. The ultrasonic atomizer according to claim 1, wherein:
the energy storage branch comprises a first capacitor;
a first end of the first capacitor is separately connected to the first boost branch, the first switch branch, and the ultrasonic atomization sheet; and
a second end of the first capacitor is connected to the ground.

6. The ultrasonic atomizer according to claim 5, wherein a capacitance value of the first capacitor is less than or equal to 100 nF.

7. The ultrasonic atomizer according to claim 1, wherein the control circuit further comprises:
a preprocessing branch, separately connected to the first boost branch, the first switch branch, and the ultrasonic atomization sheet, wherein the preprocessing branch is configured to: perform direct current blocking, voltage division, and filtering on the first drive signal, and output a first drive sub-signal;
a rectifier branch, connected to the preprocessing branch, wherein the rectifier branch is configured to rectify the first drive sub-signal, so that the energy storage branch stores energy in response to the first drive sub-signal and outputs the first detection voltage; and
a voltage-limiting and current-limiting branch, separately connected to the rectifier branch, the energy storage branch, and the controller, wherein the voltage-limiting and current-limiting branch is configured to: perform voltage-limiting and current-limiting on the first detection voltage, and output a second detection voltage to the controller, so that the controller determines the current working state of the ultrasonic atomizer based on the second detection voltage.

8. The ultrasonic atomizer according to claim 7, wherein:
the preprocessing branch comprises a second capacitor, a third capacitor, a third resistor, and a fourth resistor;
a first end of the second capacitor is separately connected to the first boost branch, the first switch branch, and the ultrasonic atomization sheet;
a second end of the second capacitor is connected to a first end of the third resistor;
a second end of the third resistor is separately connected to a first end of the third capacitor, a first end of the fourth resistor, and the rectifier branch; and
a second end of the third capacitor and a second end of the fourth resistor are connected to the ground.

9. The ultrasonic atomizer according to claim 7, wherein:
the rectifier branch comprises a first diode and a fifth resistor;
an anode of the first diode is connected to the preprocessing branch;
a cathode of the first diode is connected to a first end of the fifth resistor; and
a second end of the fifth resistor is separately connected to the energy storage branch and the voltage-limiting and current-limiting branch.

10. The ultrasonic atomizer according to claim 7, wherein:
the voltage-limiting and current-limiting branch comprises a sixth resistor and a seventh resistor;
a first end of the sixth resistor is separately connected to a first end of the seventh resistor, the rectifier branch, and the energy storage branch;
a second end of the sixth resistor is connected to the ground; and
a second end of the seventh resistor is connected to the controller.

11. The ultrasonic atomizer according to claim 1, wherein the control circuit further comprises:
a drive branch, separately connected to the controller, the first switch branch, and the power supply, wherein:
the drive branch is configured to output a second pulse signal in response to a current outputted by the power supply and the first pulse signal; and
a drive capability of the second pulse signal is stronger than that of the first pulse signal.

12. The ultrasonic atomizer according to claim 11, wherein:
the drive branch is further configured to output a fourth pulse signal in response to the current outputted by the power supply and a third pulse signal outputted by the controller; and
the control circuit further comprises:
a second switch branch, connected to the drive branch, wherein the second switch branch is configured to turn on or turn off in response to the second pulse signal; and
a second boost branch, separately connected to the power supply, the second switch branch, and the ultrasonic atomization sheet, wherein the second boost branch is configured to boost the output voltage of the power supply in response to the turning-on or the turning-off of the second switch branch, to output a second drive signal for driving the ultrasonic atomization sheet.

13. The ultrasonic atomizer according to claim 12, wherein:
the second switch branch comprises a second switch, an eighth resistor, and a ninth resistor,
a first end of the second switch is connected to the drive branch;
a third end of the second switch is separately connected to the ultrasonic atomization sheet and the second boost branch;
a first end of the eighth resistor is connected to the controller;
a second end of the eighth resistor is connected to a first end of the ninth resistor; and
both a second end of the second switch and a second end of the ninth resistor are connected to the ground.

14. The ultrasonic atomizer according to claim 12, wherein:
the second boost branch comprises a second inductor;
a first end of the second inductor is connected to the power supply; and
a second end of the second inductor is separately connected to the second switch branch and the ultrasonic atomization sheet.

15. The ultrasonic atomizer according to claim 12, wherein:
the drive branch comprises a drive chip;
the drive chip comprises a power supply input end, a first signal input end, a second signal input end, a first signal output end, and a second signal output end;
the power supply input end is connected to the power supply;
both the first signal input end and the second signal input end are connected to the controller,
the first signal output end is connected to the first switch branch;
the second signal output end is connected to the second switch branch;
the first signal input end is configured to input the first pulse signal;
the first signal output end is configured to output the second pulse signal;
the second signal input end is configured to input the third pulse signal; and
the second signal output end is configured to output the fourth pulse signal.

16. The ultrasonic atomizer according to claim 1, wherein:
the control circuit further comprises a current detection branch;
the current detection branch is separately connected to the power supply, the first boost branch, and the controller; and
the current detection branch is configured to detect a current flowing into the first boost branch.

17. The ultrasonic atomizer according to claim 16, wherein:
the current detection branch comprises an amplifier and a tenth resistor;
the tenth resistor is separately connected to the amplifier, the first boost branch, and the power supply;
the amplifier is connected to the controller; and
the amplifier is configured to output a third detection voltage based on a voltage across two ends of the tenth resistor, so that the controller determines, based on the third detection voltage, the current flowing into the first boost branch.

18. A method for determining a working state of an ultrasonic atomizer, wherein the ultrasonic atomizer comprises an ultrasonic atomization sheet, and the method comprises:
obtaining, in a working process of the ultrasonic atomization sheet, a positive half or a negative half of a drive voltage driving the ultrasonic atomization sheet, and performing charge accumulation under driving of the positive half or the negative half of the drive voltage;
obtaining, if a maximum change value of a voltage obtained through the charge accumulation is less than a preset change threshold within first duration, a voltage obtained through current charge accumulation, and recording the voltage as a first detection voltage; and
determining a current working state of the ultrasonic atomizer based on the first detection voltage.

19. The method according to claim 18, wherein:
the first duration is any one of duration in (0, 10 ms]; or
the first duration is duration longer than or equal to five sampling periods, and each sampling period is any one of duration in (0, 100 µs].

20. The method according to claim 18, wherein the determining a current working state of the ultrasonic atomizer based on the first detection voltage comprises:
determining, if the first detection voltage is less than a first voltage threshold, that the current state of the ultrasonic atomizer is a normal working state; or
determining, if the first detection voltage is greater than or equal to a first voltage threshold, that the current working state of the ultrasonic atomizer is an abnormal working state.
